# EUROPEAN PATENT SPECIFICATION

(11) **EP 2 015 694 B1**
(45) Date of publication and mention of the grant of the patent: **20.10.2010**
(21) Application number: 07761025.1
(22) Date of filing: 20.04.2007
(51) Int. Cl.: A61B 17/70, A61B 17/88

(54) **ORTHOPEDIC IMPLANT APPARATUS**
ORTHOPÄDISCHES IMPLANTAT
APPAREIL D'IMPLANT ORTHOPÉDIQUE

(30) Priority: 28.04.2006 US 414832
(43) Date of publication of application: 21.01.2009
(73) Proprietor: Warsaw Orthopedic, Inc., Warsaw, Indiana 46581 (US)
(72) Inventor: REZACH, William, A., Atoka, TN 38004 (US); MILLER, Keith, E., Germantown, TN 38138 (US)
(74) Representative: Hutchinson, Glenn Stanley
(86) International application number: PCT/US2007/067099
(87) International publication number: WO 2007/127682

(56) References cited:
- EP-A- 1 190 678
- WO-A-98/12977
- WO-A-03/028566
- US-A1- 2005 192 573
- US-A1- 2006 025 768
- US-A1- 2006 111 713

## Description

This disclosure generally concerns improved orthopedic fixation devices and implants, In particular, it concerns fixation device and implants including an attaching portion for interaction with an instrument which engages and seats an elongate member in an implant receiver portion.

Several techniques and systems have been developed for correcting and stabilizing the spine and for facilitating fusion at various levels of the spine. In one type of system, a bendable elongate member, such as a rod, is disposed longitudinally along the length of the spine or vertebral column. The elongate member may be bent to correspond to the normal curvature of the spine in the particular region being instrumented. For example, the elongate member can be bent to form a normal kyphotic curvature for the thoracic region of the spine, or a lordotic curvature for the lumbar region. In accordance with such a the elongate member can be engaged to various vertebrae along the length of the spinal column by way of fixation implants. A variety of fixation implants can be provided which are configured to engage specific portions of the vertebra. For instance, one such fixation implant is a hook that is configured to engage the laminae of the vertebra. Another very prevalent fixation implant is a spinal screw which can be threaded into various aspects of the vertebral bone.

Commonly, the fixation implants and the elongate member(s) are placed separately, that is, they are not connected together prior to implantation in the body. For example, bone screws may be implanted into vertebrae first, connectors may be placed on or around the screws (if necessary), and then the elongate member may be placed into the body. The elongate member may be contoured prior to insertion to approximate the curvature desired, or it may be contoured after placement adjacent the spine. In cases where an elongate member and fixation implant are separately placed, the elongate member and fixation implant may be required to be forced toward each other for connection. The process of moving the elongate member and fixation implant toward each other for connection is generally termed "reduction."

US 2005/0192573 discloses a peptide screw assembly and method of assembly comprising a longitudinal member; a screw head comprising a bulbous end, wherein the screw head has a slot adapted to receive the longitudinal member and indent features on the outside of the screw head; a bone fixator component comprising a concave socket having a biased angled top and a rounded bottom adapted to receive the screw head; a locking pin adapted to engage the screw head, the bone fixator component, and the longitudinal member; and a blocker adapted to engage the screw head and to secure the longitudinal member.

US 2006/0025768 is directed to a top loading spinal fixation device and instruments for loading and handling the same, wherein the device comprises a top end, a bottom end, and a pair of side walls connected at the bottom end defining an elongated U-shaped channel having a first longitudinal axis and a top opening located at the top end. The device further comprises recesses configured to communicate with the U-shaped channel thereby forming an angle with respect to the longitudinal axis of the U-shaped channel.

EP 1 190 678 is directed to a lock cap anchor assembly for orthopaedic fixation, comprising a top member with an open slot to receive a linkage such as a rod, and a twist-lock closure cap to close the open and, capturing the linkage in the slot. One closure cap fits over and around the top member, with a set of segmented protrusions that extend through a corresponding set of protruding flange segments spaced about the circumference of the top member. The cap is configured to rotate and lock against the top member like a flange-locking bayonet mount when turned through a limited degree of rotation.

WO 03/028566 is directed to a spinal osteosynthesis assembly comprising the head of an anchoring member consisting of at least one arm and a tool for fixing said head to a spinal osteosynthesis system. The head comprises an edge presenting a chamfer extending from the tip of the edge to a low portion of the head where it extends in continuity with the corresponding edge of the other branch. Each pair of edges thus forms a U-shape. The head presents second chamfers extending to the junction of each edge with the inside faces of the branches. This second chamfer is also generally U-shaped. It faces essentially towards the inside of the head. Each lateral edge of each branch presents a blind cavity formed both in the edge and in the second chamfer, cutting into the ridge formed at the junction thereof. Each cavity presents a plane end face which constitutes the largest face of the cavity. The cavity opens out laterally relative to the edge and the second chamfer. In contrast, it is closed upwards and downwards by residual portions of the ridge.

Reduction can be accomplished by hand, although the environment and close quarters of a surgical site can make reduction by hand quite difficult. Instruments have been developed to provide a mechanical advantage in reducing or positioning the elongate member relative to an implant. There remains a need for improved fixation implants that aid in reducing or seating an elongate member.

### SUMMARY OF THE INVENTION

The present invention provides a medical implant device as defined in claims 1 to 14.

### BRIEF DESCRlPTION OF THE DRAWINGS

FIG. 1 is a perspective view of one embodiment of an orthopedic implant.
FIG. 2 is a perspective view of part of the embodiment illustrated in FIG. 1.
FIG. 3 is a side elevational view of part of the embodiment illustrated in FIG. 1.
FIG. 4 is a side elevational view of part of the embodiment illustrated in FIG. 1, rotated 90 degrees from the view shown in FIG. 3.
FIG. 5 is a side view of an embodiment of an implant in relation to an elongate member and art embodiment of a reducing instrument.
FIG. 5A is a close-up view of a portion of the embodiment of a reducing instrument shown in FIG. 5.
FIG. 6 is a side view of the embodiment shown in FIG. 5 showing elongate member being reduced.
FIG. 7 is a side view of the embodiment shown in FIG. 5 showing a reduced elongate member.
FIG. 8 [not of the invention] is a side elevational view of another embodiment of an orthopedic implant.
FIG. 9 [not of the invention] is a top plan view of the embodiment shown in FIG. 8.

### DESCRIPTION OF THE ILLUSTRATED EMBODIMENT

For the purposes of promoting an understanding of the principles of the disclosure, reference will now be made to the embodiment illustrated in the drawings and specific language will be used to describe the same. It will nevertheless be understood that no limitation of the scope of the claims is thereby intended, such alterations and further modifications in the illustrated device, and such further applications of the principles of the disclosure as illustrated therein being contemplated as would normally occur to one skilled in the art to which the disclosure relates.

Referring generally to the figures, there is shown an embodiment of an orthopedic implant 20. In the illustrated embodiment, implant 20 is characterised by an anchoring portion 22 as described in greater detail below. Described herein to a receiving portion 24 of the implant having two branches 25 defining a channel 26 for receiving an elongate member 28, such as, but not limited to, a spinal rod, bar or similar apparatus. Receiving portion 24 also includes at least one attaching portion (e.g. 30), located on a branch 25 at or adjacent a side (e.g. 32) of receiving portion 24. Receiving portion 24 may include, additional attaching portions. In the illustrated embodiment, receiving portion 24 includes four attaching portions 30, 36, 37, and 38, two of which are at or adjacent side 32 and two of which are at or adjacent side 33. Thus, attaching portions 30, 36, 37 and 38 are closer to one side 32 or 33 than they are to the other. Attaching portion(s) 30, 36, 37, and/or 38 provide for engagement of a reducing instrument 39, which then aids in the seating or reducing of elongate member 28 in channel 26 of implant 20.

Anchoring portion 22 may be made for attachment to bone, such as cervical, thoracic, lumbar and or sacral vertebral bone structures, or other tissues. Anchoring portion 22 may be a screw, or could also be alternatively configured, for example as a vertebral hook, clamp, or other structure. In the illustrated embodiment, anchoring portion 22 is of a monoaxial bone screw variety, having a shaft 40 with one or more threads 41 on at least a portion of shaft 40, e.g. a relatively lower portion. Thread 41 may be a cancellous thread, of a configuration suited to implantation into a vertebra or similar bone. Thread 41 may be self-tapping or intermittent, or may have more than one crest winding about anchoring portion 22, or of other appropriate configurations. A neck 42 at a relatively upper portion of anchoring portion 22 is provided. Neck 42 may be above thread 41, or may also include thread(s) or a threaded portion, In an embodiment in which anchoring portion 22 is integrally joined to receiving portion 24, as by unitary formation or construction or by solid attachment, neck 42 is attached to receiving portion 24.

Receiving portion 24 is substantially U-shaped in the illustrated embodiment, with an intermediate portion 50 substantial between branches 25 and adjacent sides 32 and 33. Sides 32, 33 may be generally vertical (i.e., generally parallel to shaft 40 of the illustrated embodiment), for a "top loading" implant 20. It will be seen that sides 32, 33 could also be generally horizontal (i.e. generally perpendicular to shaft 40), for a "side-loading" implant 20, or otherwise angled with respect to other parts of implant 20. Examples of side-loading implants are disclosed in U.S. Patent App. Serial Nos. 11/000,585 (US 2006/0116687), filed November 30,2004 and 11/000,846 (US 2006/0116677), filed December 1, 2004 and entitled, respectively, SIDE-LOADING ADJUSTABLE BONE ANCHOR and SIDE-LOADING BONE ANCHOR.

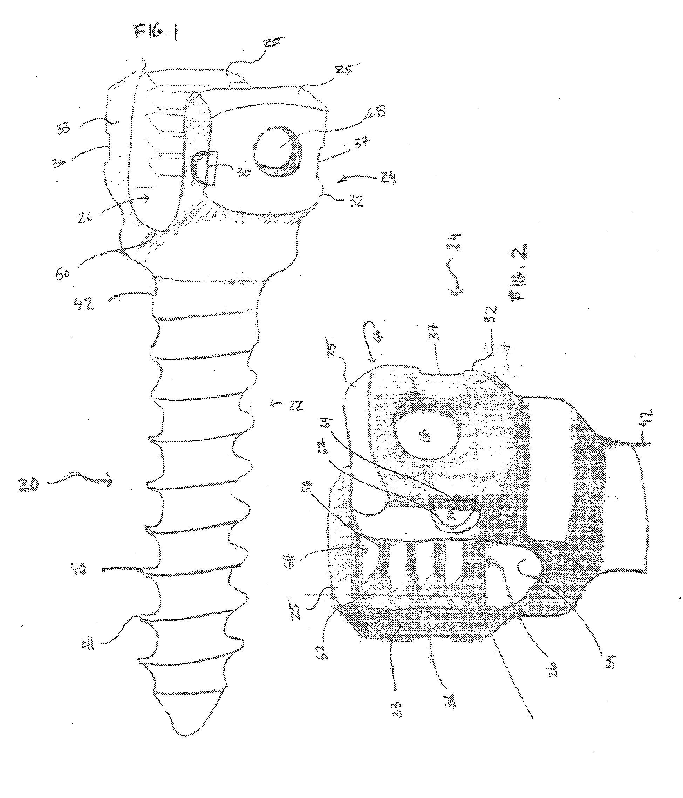

Receiving portion 24 may be tapered at intermediate portion 50 where it meets the anchoring portion 22, which aids in reducing the overall bulk of implant 20. In the illustrated embodiment, neck 42 is integral with receiving portion 24. As shown in FIG. 1, neck 42 may be attached in a particular embodiment to receiving portion 24 via intermediate portion 50. Neck 42 may also be connected to receiving portion 24 at other parts of receiving portion 24. As a non-limiting example, neck 42 could be connected to one branch 25, with intermediate portion 50 and another branch 25 being offset to one side of the anchoring portion 22. Further, implant 20 could include a receiving portion 24 which is multi-axial, pivotable or otherwise adjustable with respect to anchoring portion 22, such as disclosed in U.S. Patent No. 6,280,442, issued August 28, 2001 and entitled MULTI-AXIAL BONE SCREW ASSEMBLY.

In the illustrated embodiment, intermediate portion 50 joins branches 25. Taken together, branches 25 and intermediate portion 50 form a general U-shape, with a channel 26 substantially above intermediate portion 50. Channel 26 is shaped and sized to be compatible with elongate member 28; channel 26 could have a slightly smaller circumference to aid in retaining elongate member 28 through a compression-fit or "wedging" action. Additionally, channel 26 and elongate member 28 could be shaped in a nesting or other configuration so as to allow elongate member 28 to be connected to implant 20 and reduce any slipping action.

In the illustrated embodiment, channel 26 has a bottom surface 54 that may be substantially continuous, bifurcated or otherwise divided, as well as internal threads 58 that can accommodate an externally-threaded set screw 59. In one particular embodiment, internal threads 58 and/or the thread on set screw 59 may be a reverse-angle thread, as disclosed in U.S. Patent No. 6,296,642. In other embodiments, channel 26 might be rectangular, triangular, or otherwise polygonal. Further, implant 20 could have multiple channels.

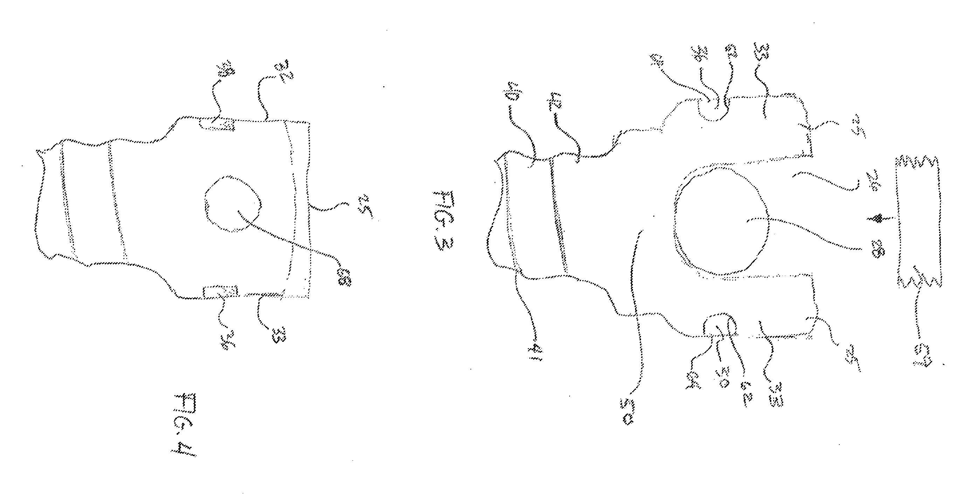

As shown in FIGS. 2 and 3, the illustrated embodiment of receiver portion 24 includes a pair of opposed sides 32, 33, and each branch 25 includes an outer surface 60. Attaching portions 30,36,37,38 are in or on branches 25, at or adjacent to outer surface 60. Thus, in this embodiment attaching portion 30 is on side 32 of one branch 25, and attaching portion 37 is on side 32 of that same branch 25. Attaching portions 36 and 37 are on sides 32 and 33 of the other branch 25, respectively. Each attaching portion is substantially identical to the others in the illustrated embodiment, and so for clarity and brevity attaching portion 30 will be described in detail. Attaching portion 30 is an aperture or indentation having part- or semi-circular portion 62 and an opening 64. In this particular embodiment, all or substantially all of attaching portion 30 is on or at side 32, and opening 64 is in or adjacent outer surface 60. Outer surface 60 may further include a separate hole 68 for accommodating a tool (not shown) for gripping or turning implant 20.

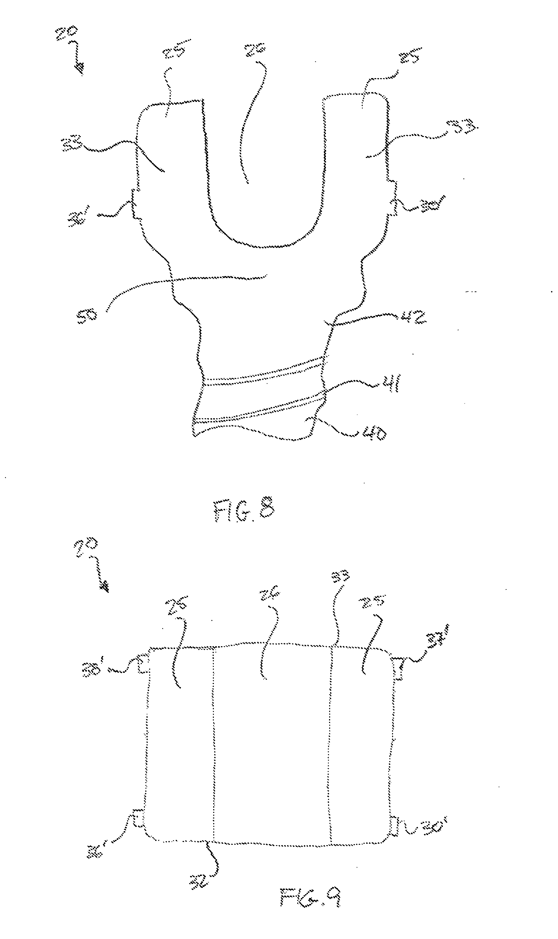

In other embodiments, an attaching portion such as attaching portion 30 may be of other types or shapes of aperture, such as an indent, slot, or bore, and may be open (like attaching portion 30 with its opening 64) or closed. An attaching portion such as attaching portion 30 could also be may be single or multiple protrusions, such as a boss. Thus, as seen in the embodiment of apparatus 20 seen in FIGS. 8-9 [not of the invention], attaching portions 30', 36', 37', and 38' include substantially circular protrusions 62'. It will be seen that although the illustrated embodiments show four attaching portions that are alike, in other embodiments a different number of non-alike types of attaching portions may be used. An attaching portion (e.g. 30) should be generally configured to allow relatively simple engagement with a portion of an instrument such as reducing instrument 39. Such engagement allows the instrument 39 to pivot or rotate with respect to the receiving portion 24, thereby enabling reduction of an elongate member 24. Further, in the illustrated embodiments attaching portions 30, 36, 37 and 38 are located approximately halfway between the top of receiving portion 24 and bottom surface 54 of channel 26. It will be seen that one or more attaching portions could be differently placed, e.g. nearer to surface 54 (e.g. portions 30', 36', 37', 38' of FIGS, 8-9) or to the top of receiving portion 24. Attaching portions on the same branch 25 (e.g. attaching portions 30 any 37) need not be placed at the same depth along channel 26.

In the illustrated embodiment, where channel is U-shaped, attaching portions 30, 36, 37, 38 are located substantially at the intersections of outer surface 60 and sides 32, 33 of each branch 25. Thus, attaching portions 30, 36, 37, 38 are each at a side 32 or 33, or are at least nearer to one side 32 or 33 than to the other side 32 or 33. Attaching portions 30, 36, 37, 38 are offset from channel 36 in the illustrated embodiments, but could be located under a portion of channel 26, e.g. in intermediate portion 50 of receiving portion 24. Pivoting motion of an instrument 39 can occur perpendicular to the plane or branches, thereby providing a motion for an elongate member 28 which lies offset from channel 26 to be moved to or within channel 26. This pivoting action with respect to the instrument 34, implant 20 and elongate member 28 is discussed more fully bellow with respect to operation of the instrument 34 and implant 20.

In the illustrated embodiment, outer surface 60 includes a gripping portion 68 in the term of an indentation. The gripping portion 68 is configured to receive a gripping or positioning tool. Although shown in the illustrated embodiment as a circular indentation, gripping portion could take the form of a single or multiple slot(s), hole(s), boss(es), any combination of these, or other forms as would occur to a person having ordinary skill in the art. The gripping portion generally allows the surgeon to grip the receiving portion 24 with an appropriate tool (not shown) having one of more rounded or circular protrusions or indentations at or adjacent to the end(s) of such a tool. In conjunction with an appropriate tool, the gripping portion 68 may be used to hold, position, manipulate or otherwise work on or with the implant 20. In the illustrated embodiment, there is a gripping portion 68 on each branch 25, in an approximately central location in outer surface 60.

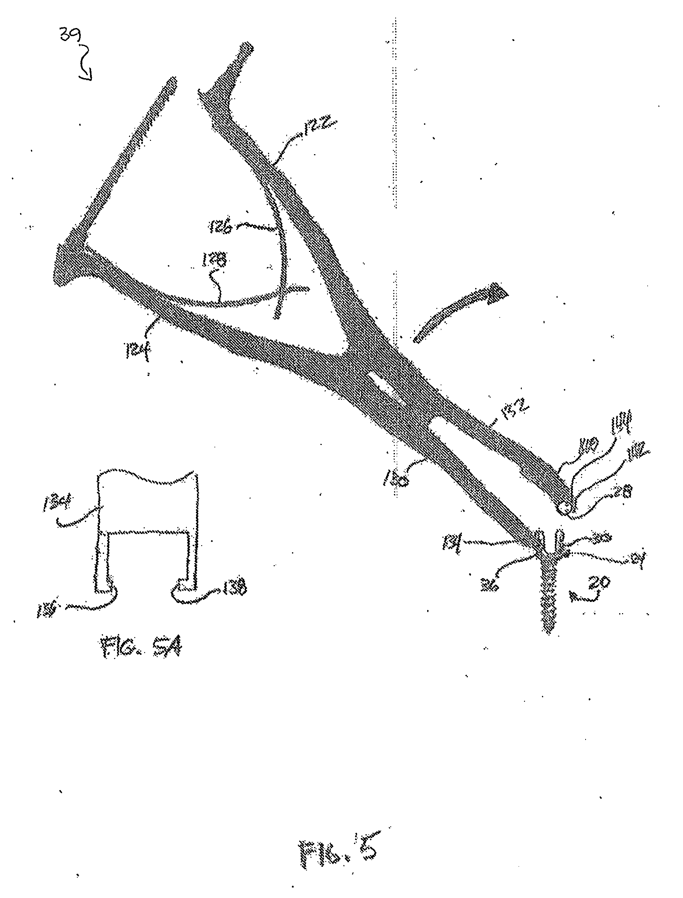

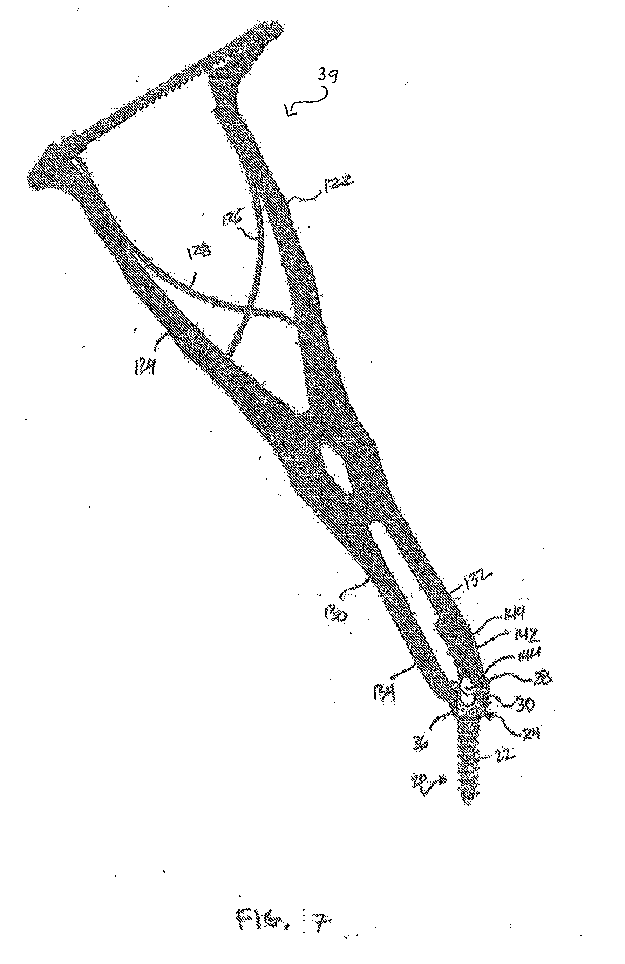

Implant 20 is configured to removably engage, via one or more attaching portions (e.g. 30 and 37), a reducing instrument 39. Instrument 39 [not of the invention] is operable to move an elongate member 28 and the implant 20 toward each other. An embodiment of instrument 39 is illustrated in FIGS. 5-7. Reducing instrument 39 includes handle portions 122 and 124, springs 126 and 128, and arm portions 130 and 132. Distal end 134 of arm 130 has extension portions 136 and 138. Extension portions 136 and 138 may be substantially circular protrusions, in one embodiment, that are insertable into the embodiment of attaching portions 30, 36, 37, 38 described above. Distal end 140 of arm 132 includes a rod-contacting portion 142 that may have a groove 144 for accommodating a part of elongated member 28.

In using the illustrated embodiment of instrument 39, it will be seen that squeezing handle portions 122 and 124 together causes rotation of handle portions 122 and 124 with respect to each other, so that their respective distal ends and the proximal parts of arms 130 and 132 move apart. When the proximal parts of arms 130 and 132 move apart, their respective distal portions 134 and 140 move together. Thus, by squeezing handle portions 122 and 124 together, the distal portions 134 and 140 are forced together.

Elongate member 28, in the illustrated embodiment, is substantially cylindrical and generally configured to fit within channel 26, and may have a shape and size similar or substantially identical to the shape or size of channel 26. For example, where channel 26 is substantially U-shaped, elongate member 28 may be circular (to mate with the bottom of the U-shaped channel 26).

In other embodiments, elongate member 28 may have a shape different from that of channel 26. As one example, if channel 26 is substantially cylindrical, elongate member 28 may be square, with a diagonal length at least slightly greater than the diameter of channel 26, so that corners of such an insert member will firmly engage one or more wall(s) of channel 26. Where elongate member 28 and channel 26 are each substantially cylindrical, the diameter of elongate member 28, or some part of it, may be slightly lager than the diameter of channel 26. Elongate member 28 may also have portions of its surface roughened or otherwise modified to introduce friction between elongate member 28 surface and wall(s) of channel 26, thereby further stabilizing the connection between implant 20 and elongate member 28. Elongate member 28 may be made of any sturdy biocompatible material, such as metals or other biocompatible materials which are somewhat flexible to allow elongate member 28 to be connected to various fixtures, such as implant 20.

The operation of instrument 39 to engage an implant 20 and elongate member 28 and seat the elongate member 28 in the implant 20 receiving portion 24 will now be described with respect to operation on a spinal column. Alternative uses with respect to other bony structures or other tissues can be made. As with other types of orthopedic surgery, an incision is made and access is gained to the surgical site. The approach to the surgical site can be an open approach, i.e. a relatively long incision with retraction of underlying tissue. The implant 20 and/or instrument 39 disclosed herein can be used in such an approach, or with other surgical techniques.

After access to the surgical site has been obtained, a fixation implant 20 including a receiving portion 24 is inserted into bone or other tissue able to accommodate the anchoring potion 22. Implant 20 may have a unitary construction or be pre-fitted with receiving portion 24. Channel 26 of receiving portion 24 may, as shown in FIGS 1-3. open to the top, or point substantially to the side or may open to the back of the implant 20, or be otherwise oriented. As discussed above, receiving portion 24 may be poly-axial, pivotable or otherwise adjustable with respect to anchoring portion 22, and, if so, receiving portion 24 could be placed on or over anchoring portion 22 after engagement of the anchoring portion 22 into bone. An elongate member 28, is inserted into the surgical site, and placed adjacent one or more implant(s) 20. If not already present, receiving portion 24 may be loosely placed on the anchoring portion 22 prior to insertion of the elongate member 28 to the surgical site. The implant 20 and elongate member 28 are manipulated so that a part of the elongate member 28 is near each of the implants.

Once the surgeon has positioned the elongate member 28 as close to an implant. 20 as is reasonably possible, instrument 39 may be introduced to reduce or force the elongate member 28 into the implant 20. With handle portions 122 and 124 in an unstressed state, i.e. biased away from each other or otherwise spread apart, distal ends 134 and 140 of arm portions 130 and 132 are also spread apart. Distal ends 134 and 140 are placed around the combination of elongate member 28 and receiving portion 24, so that distal end 140 is adjacent to of abutting a surface of elongate member 28 relatively distant from receiving portion 24, and distal end 134 is adjacent to or abutting a surface of receiving portion 24 relatively distant from elongate member 28. In the illustrated embodiment, extensions 136 and 138 of instrument 39 are inserted into a pair of attaching portions (e.g. 30 and 37) of implant 20. For example, extensions 136 and 138 may be maneuvered through respective openings 64 of attaching portions 30 and 37 and placed adjacent to or engaging respective surface 62 of those attaching portions. As discussed above, attaching portion 30 may be a protrusion, slot or indentation and attaching portion 262 is formed to mate with or otherwise engage attaching portion 30. In an alternative embodiment in which receiving portion 24 includes one or more protrusions or bosses (e.g. one or more of 30', 36', 37', 38') rather than hollow(s), distal end 134 may be provided with one or more hollows in place of extensions 136 and/or 138 to accommodate such protrusions.

When engaged, instrument 39 can pivot or rotate with respect to receiving portion 24 of implant 20 substantially around an axis through extensions 136 and 138 and through the attaching portions (e.g. 30 and 37) to which extensions 136 and 138 are connected, as seen in one example in FIGS. 5-7. In that example, a distal portion of arm 132 lies outside the plane of channel 26. Handle portions 122 and 124 can be squeezed to push elongated member 28 toward the implant 20.

Alternatively or in addition, rotation of instrument 39 as described above in a clockwise direction (as seen in FIGS. 5-7) pushes elongated member 28 toward receiver member 24 and channel 26. In many cases, the elongate member 28 will all or substantially all of such relative movement, and the implant 20 (which is anchored) will remain relatively stationery. However, it will be appreciated that in some uses, e.g. in some cases of significant vertebral misalignment, the surgeon would prefer implant 20 and anchoring structure (e.g. bone) to undergo movement toward the elongate member 28 and thus instrument 39 can cause such movement of the implant 20, perhaps with direct manipulation of the bone or other anchoring structure by the surgeon.

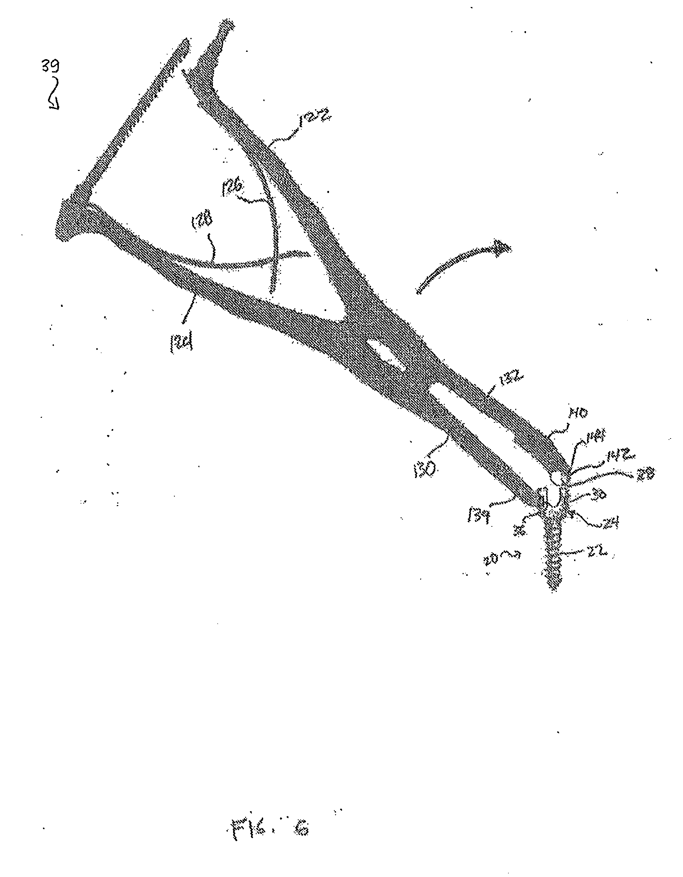

As shown in FIG. 6, pivoting of the instrument 39 and/or squeezing of handles 122, 124 may be continued until elongated member 28 is seated within receiving portion 24. The axis of rotation in that example substantially corresponds with extensions 136 and 138 and attaching portions 30 and 37, in the illustrated embodiment, it is different from the axes around which arms 122, 124, 130 and 132 pivot, and it is substantially perpendicular to the axis of elongated member 28, and the axis of channel 26 along which elongated member runs. Such pivoting may force elongate member 28 generally obliquely with respect to a longitudinal axis of implant 20, or in a direction than has at least a component parallel to a longitudinal axis of implant 20. Such pivoting or rotation can occur in this embodiment as may be necessary for reduction of elongate member 28.

These figures are only intended to detail a manner in which elongate member 28 may be seated in receiving portion 24 of implant 20. This example is not meant to be limiting, as pivoting of an instrument such as instrument 39 with respect to implant 20 and squeezing of bandies 122 may be performed multiple times and in any order necessary to achieve the effect desired by the surgeon. In particular, through such squeezing of instrument 39 and/or pivoting of instrument 39 with respect to receiving portion 24, or a combination of the two motions, elongate member 28 is reduced into channel 26 of receiving portion 24.

Once the elongate member 28 is positioned as the surgeon desires in the implant 20, the elongate member 28 can be locked into the implant using a locking member 59 (e.g. set screw, cap, clamp) provided with the implant 20.

Instrument 39 may be removed from contact with the elongate member 28 and the implant 20 after such locking, or before if the elongate member 28 will remain at least approximately in the position desired by the surgeon. The surgeon may then seat or reduce the elongate member 28 with respect to the receiving portion 24 of any additional implants, thereby connecting the elongate member 28 to als many implants (implants 20 or other variety of implant) as the operation requires. The surgeon may also remove instrument 39 from the surgical site to perform other tasks and then return to seating of additional implants and/or elongate member or conclude the surgical procedure.

As described above, the implant(s) 20 and elongate member (s) 28 may be positioned in or along one or more parts of the spine, including the cervical, thoracic, lumbar and/or sacral portions. Although the use of the embodiment of implant 20 has been described in the above context, this embodiment and others could be used with a variety of screws, hooks or other fixation implants, or in connection with orthopedic instruments and elongate member(s) in parts of the body other than the spine. It will be seen that fewer than four attaching portions may be provided on receiving portion 24. For example, only two attaching portions may be provided (e.g. 30 and 36) on one branch 25 of receiving portion 24, In other embodiments, at least one attaching portion may be provided to permit the pivoting motion described above.

The above embodiment may be made of stainless steel, certain hard plastics, or other materials that are compatible with surgical procedures and the elongate members and instruments with which it is used.

While the disclosure has been illustrated and described in detail in the drawings and foregoing description, the same is to be considered as illustrative and not restrictive in character.

## Claims

1. A medical implant device, comprising:
an anchoring portion (22) for connection to a bone or a vertebra; and
a receiving portion (24) comprising:
a first side (33);
a second side (32);
a channel (26) extending along an axis and passing from said first side (33) to said second side (32), said channel (26) adapted to receive an elongate member (28) passing from said first side (33) to said second side (32);
an outer surface (60) extending axially along said channel (26) and between said first (33) and second (32) sides; and
at least one attaching portion (30), wherein said attaching portion (30) is **characterised in that** it comprises a partially cylindrical recess located at an intersection between said outer surface (60) and said first side (33), wherein said recess extends axially into said first side (33) to form an arcuate aperture (62) in said first side (33) and defines an opening (64) on said outer surface (60).

2. The device of claim 1, wherein said arcuate aperture (62) is semi-circular.

3. The device of claim 1 or claim 2, further comprising a second attaching portion on said receiving portion (24).

4. The device of claim 3, wherein said first (30) and said second (36) attaching portions are each located closer to the same one of said sides than to the other.

5. The device of claim 4, wherein said first (30) and said second (36) attaching portions are located substantially at the same side.

6. The device of claim 3, wherein said first attaching portion (30) is located substantially at said first side (33) and said second attaching portion (37) is located substantially at said second side (32).

7. The device of claim 4, further comprising a third and fourth attaching portions, said first and second attaching portions (30, 36) located nearer said first side (33) than said second side and said third and fourth attaching portions (37, 38) located nearer said second side (32) than said first side.

8. The device of any preceding claim , wherein said receiving portion comprises a first and second branch (25) defining said channel (26) therebetween, wherein said outer surface (60) is positioned on each of said first and second branches, axially along said channel.

9. The device of claim 8, wherein said first or second branch (25) comprises a gripping portion (68) for receiving a gripping or positioning tool.

10. The medical device of claim 9, wherein the gripping portion (68) is in an approximately central location in the outer surface (60) of the branch (25).

11. The device of claim 9 or claim 10, wherein the gripping portion (68) is a circular indentation, a slot, a hole, a boss, or a combination thereof.

12. The device of any preceding claim, wherein said attaching portion or portions (36, 38) are configured to engage with an extension or extensions (136, 138) of a reducing instrument (39).

13. The device of claim 12, wherein said attaching portion or portions (36, 38) allow the reducing instrument (39), when engaged with said attaching portion or portions (36, 38), to pivot or rotate with respect to the receiving portion (24) substantially around an axis through said extension or extensions (136, 138).

14. The device of claim 12, wherein said attaching portion or portions (36, 38) allow the reducing instrument (39), when engaged with said attaching portion or portions (36, 38), to pivot or rotate with respect to the receiving portion (24) substantially around an axis perpendicular to said channel (26) axis.

## Patentansprüche

1. Medizinische Implantatvorrichtung, die folgendes umfasst:
ein Verankerungsteilstück (22) zur Verbindung mit einem Knochen oder einem Wirbel; und
einem Aufnahmeteilstück (24), das folgendes umfasst:
eine erste Seite (33);
eine zweite Seite (32);
einen Kanal (26), der sich entlang einer Achse erstreckt und von der genannten ersten Seite (33) zu der genannten zweiten Seite (32) verläuft, wobei der genannte Kanal (26) ein elongiertes Element (28) aufnehmen kann, das von der genannten ersten Seite (33) zu der genannten zweiten Seite (32) verläuft;
eine äußereOberfläche (60), die sich axial entlang dem genannten Kanal (26) und zwischenden genannten ersten (33) und zweiten (32) Seiten erstreckt; und
mindestens ein Befestigungsteilstück (30), wobei das genannte Befestigungsteilstück (30) **dadurchgekennzeichnet** ist, dass es einen teilweise zylindrische Aussparung umfasst, die an einer Schnittstelle zwischender genannten äußeren Oberfläche (60) und der genannten ersten Seite (33) angeordnet ist, wobei sich die genannte Aussparung axial in die genannte erste Seite (33) erstreckt, so dass eine bogenförmige Öffnung (62) in der genannten ersten Seite (33) gebildet wird und eine Öffnung (64) an der genannten äußeren Oberfläche (60) definiert wird.

2. Vorrichtung nach Anspruch 1, wobei die genannte bogenförmige Öffnung (62) halbkreisförmig ist.

3. Vorrichtung nach Anspruch 1 oder 2, wobei diese ferner ein zweites Befestigungsteilstück an dem genannten Aufnahmeteilstück (24) umfasst.

4. Vorrichtung nach Anspruch 3, wobei die genannten ersten (30) und die genannten zweiten (36) Befestigungsteilstücke jeweils näher zu denen auf der gleichen der genannten Seiten als zu den anderen angeordnet sind.

5. Vorrichtung nach Anspruch 4, wobei die genannten ersten (30) und die genannten zweiten (36) Befestigungsteilstücke im Wesentlichen auf der gleichen Seite angeordnet sind.

6. Vorrichtung nach Anspruch 3, wobei das genannte erste Befestigungsteilstück (30) im Wesentlichen auf der genannten ersten Seite (33) angeordnet ist, und wobei das genannte zweite Befestigungsteilstück (37) im Wesentlichen auf der genannten zweiten Seite (32) angeordnet ist.

7. Vorrichtung nach Anspruch 4, wobei diese ein drittes und viertes Befestigungsteilstück umfasst, wobei die genannten ersten und zweiten Befestigungsteilstücke (30, 36) näher an der genannten ersten Seite (33) als an der genannten zweiten Seite angeordnet sind, und wobei die genannten dritten und vierten Befestigungsteilstücke (37, 38) näher an der genannten zweiten Seite (32) als an der genannten ersten Seite angeordnet sind.

8. Vorrichtung nach einem der vorstehenden Ansprüche, wobei das genannte Aufnahmeteilstück einen ersten und zweiten Zweig (25) umfasst, welche dazwischen den genannten Kanal (26) definieren, wobei die genannte äußereOberfläche (60) an jedem der genannten ersten und zweiten Zweige axial entlang dem genannten Kanal positioniert ist.

9. Vorrichtung nach Anspruch 8, wobei der genannte erste oder zweite Zweig (25) ein Greifteilstück (68) zur Aufnahme eines Greif- oder Positionierungsinstruments umfasst.

10. Medizinische Vorrichtung nach Anspruch 9, wobei sich das Greifteilstück (68) an einer ungefährzentralen Position in der äußeren Oberfläche (60) des Zweigs (25) befindet.

11. Vorrichtung nach Anspruch 9 oder Anspruch 10, wobei es sich bei dem Greifteilstück (68) um eine runde Vertiefung, einen Schlitz, ein Loch, einen Vorsprung oder eine Kombination dieser Optionen handelt.

12. Vorrichtung nach einem der vorstehenden Ansprüche, wobei das genannte Befestigungsteilstück oder die genannten Befestigungsteilstücke (36, 38) so konfiguriert ist bzw. sind, dass es bzw. sie mit einer Verlängerung oder Verlängerungen(136, 138) eines Reduzierungsinstruments (39) eingreift bzw. eingreifen.

13. Vorrichtung nach Anspruch 12, wobei das genannte Teilstückoder die Teilstücke(36, 38) es ermöglichen, wenn das Reduzierungsinstrument (39) mit dem Befestigungsteilstück oder den Befestigungsteilstücken (36, 38) eingreift, dass sich das Reduzierungsinstrument im Verhältnis zu dem Aufnahmeteilstück (24) im Wesentlichen um eine zu der Achse durch die genannte Verlängerung oder die Verlängerungenschwenkt oder dreht.

14. Vorrichtung nach Anspruch 12, wobei das genannte Teilstückoder die Teilstücke (36, 38) es ermöglichen, wenn das Reduzierungsinstrument (39) mit dem Befestigungsteilstück oder den Befestigungsteilstücken (36, 38) eingreift, dass sich das Reduzierungsinstrument im Verhältnis zu dem Aufnahmeteilstück (24) im Wesentlichen um eine zu der Achse des genannten Kanals (26) senkrechte Achse schwenkt oder dreht.

## Revendications

1. Dispositif d'implant médical, comprenant :
une partie d'ancrage (22) pour la connexion à un os ou une vertèbre ; et
une partie réceptrice (24) comprenant :
un premier côté (33) ;
un second côté (32) ;
un canal (26) s'étendant le long d'un axe et passant dudit premier côté (33) audit second côté (32), ledit canal (26) étant adapté pour recevoir un élément allongé (28) passant dudit premier côté (33) audit second côté (32) ;
une surface extérieure (60) s'étendant axialement le long dudit canal (26) et entre lesdits premier (33) et second (32) côtés ; et
au moins une partie de fixation (30), dans lequel ladite partie de fixation (30) est **caractérisée en ce qu'**elle comprend un retrait partiellement cylindrique situé au niveau d'une intersection entre ladite surface extérieure (60) et ledit premier côté (33), dans lequel ledit retrait s'étend axialement dans ledit premier côté (33) pour former une ouverture arquée (62) dans ledit premier côté (33) et définit une ouverture (64) sur ladite surface extérieure (60).

2. Dispositif selon la revendication 1, dans lequel ladite ouverture arquée (62) est semi-circulaire.

3. Dispositif selon la revendication 1 ou 2, comprenant en outre une deuxième partie de fixation sur ladite partie réceptrice (24).

4. Dispositif selon la revendication 3, dans lequel lesdites première (30) et deuxième (36) parties de fixation sont chacune situées plus près du même desdits côtés que de l'autre.

5. Dispositif selon la revendication 4, dans lequel lesdites première (30) et deuxième (36) parties de fixation sont situées sensiblement du même côté.

6. Dispositif selon la revendication 3, dans lequel ladite première partie de fixation (30) est située sensiblement au niveau dudit premier côté (33) et ladite deuxième partie de fixation (37) est située sensiblement au niveau dudit second côté (32).

7. Dispositif selon la revendication 4, comprenant en outre des troisième et quatrième parties de fixation, lesdites première et deuxième parties de fixation (30, 36) étant situées plus près dudit premier côté (33) que dudit deuxième côté et lesdites troisième et quatrième parties de fixation (37, 38) étant situées plus près dudit second côté (32) que dudit premier côté.

8. Dispositif selon l'une quelconque des précédentes revendications, dans lequel ladite partie réceptrice comprend une première et une seconde branches (25) définissant ledit canal (26) entre elles, dans lequel ladite surface extérieure (60) est positionnée sur chacune desdites première et seconde branches, axialement le long dudit canal.

9. Dispositif selon la revendication 8, dans lequel ladite première ou seconde branche (25) comprend une partie de préhension (68) pour recevoir un outil de préhension ou de positionnement.

10. Dispositif médical selon la revendication 9, dans lequel la partie de préhension (68) se trouve à un emplacement approximativement central dans la surface extérieure (60) de la branche (25).

11. Dispositif selon la revendication 9 ou 10, dans lequel la partie de préhension (68) est une indentation circulaire, une fente, un trou, un bossage ou une combinaison de ceux-ci.

12. Dispositif selon l'une quelconque des précédentes revendications, dans lequel ladite ou lesdites parties de fixation (36, 38) sont configurées pour venir en prise avec une ou plusieurs extensions (136, 138) d'un instrument de réduction (39).

13. Dispositif selon la revendication 12, dans lequel ladite ou lesdites parties de fixation (36, 38) permettent à l'instrument de réduction (39), lorsqu'il est en prise avec ladite ou lesdites parties de fixation (36, 38), de pivoter ou de tourner par rapport à la partie réceptrice (24) sensiblement autour d'un axe à travers ladite ou lesdites extensions (136, 138).

14. Dispositif selon la revendication 12, dans lequel ladite ou lesdites parties de fixation (36, 38) permettent à l'instrument de réduction (39), lorsqu'il est en prise avec ladite ou lesdites parties de fixation (36, 38), de pivoter ou de tourner par rapport à la partie réceptrice (24) sensiblement autour d'un axe perpendiculaire à l'axe dudit canal (26).
